Europäisches Patentamt

European Patent Office

Office européen des brevets

(19)

(11) Publication number: **0 203 715 B1**

(12) **EUROPEAN PATENT SPECIFICATION**

(45) Date of publication of patent specification: 06.03.91 Bulletin 91/10

(51) Int. Cl.⁵: **A61F 13/15**

(21) Application number: **86303137.3**

(22) Date of filing: **25.04.86**

(54) Wetness indicator for disposable diaper.

(30) Priority: **26.04.85 US 727477**

(43) Date of publication of application: **03.12.86 Bulletin 86/49**

(45) Publication of the grant of the patent: **06.03.91 Bulletin 91/10**

(84) Designated Contracting States: **FR GB IT**

(56) References cited:
US-A- 4 192 311
US-A- 4 231 370
US-A- 4 287 153

(73) Proprietor: **Johnson & Johnson Products Inc. 501 George Street New Brunswick New Jersey 08903 (US)**

(72) Inventor: **Boardman, Franklin 28 Bunker Hill Drive Englishtown New Jersey 07726 (US)**

(74) Representative: **Jones, Alan John et al CARPMAELS & RANSFORD 43 Bloomsbury Square London, WC1A 2RA (GB)**

EP 0 203 715 B1

Jouve, 18, rue Saint-Denis, 75001 PARIS

## Description

### Background of the Invention

The present invention relates to disposable diapers whether they be infant or adult which are adapted to be used once and then discarded. More particularly, the invention is directed to diapers which provide a convenient visible indication of their used condition and, hence, readiness to be discarded. Specifically, the invention provides a loosely-compacted, cellulosic fibrous batt as the absorbent pad, one side of which is in contact with a translucent or light-transmitting backing sheet of water-impermeable material with a water-dispersible or water-soluble indicator strategically placed on the batt.

Wetness indicators for disposable diapers, particularly adult diapers, have been provided in the art for some time. For example, U.S. Patent 2,681,032 discloses a diaper wetness indicator which is actuated on a mechanical principle. When the diaper becomes wet, a low wet strength element breaks moving a colored indicator into registry with an aperture through which the indicator is visible.

U.S. Patent 3,675,654 provides a moisture actuated indicating agent between an absorbent pad and a translucent back sheet. The indicating agent is dry so that when it becomes wet in use, it is visible through the translucent back sheet.

U.S. Patent 3,702,610 provides an indicating means in a water-dispersible adhesive composition in a predetermined pattern. As the liquid disperses the adhesive, the adhesive pattern substantially disappears. U.S. Patent 3,759,261 provides a layer of absorbent material carrying a printed pattern and at least one outer layer which is of sufficient density as to conceal the printed pattern when the diaper is dry but rendering the printed pattern readily visible when the diaper is wet. U.S. Patent 3,918,454 provides an indicator layer which is ruptured through the absorptive layer to provide contact with the liquid and, hence, conducts moisture through to the visible portion of the indicator layer. U.S. Patent 3,952,746 provides an external indicator strip which is in registry with a hole leading into the absorbent batt so that the indicator strip becomes moistened on one end and the liquid then wicks to the other end to provide the indication of diaper use.

U.S. Patent 4,022,211 provides a wetness indicator which is a water-dispersible or water soluble coloring agent affixed to a carrier means which is adjacent the absorbent pad and of sufficient intensity to be readily visible through the backing sheet when the pad is dry. After the pad is wetted, the indicator becomes substantially invisible. The carrier means is generally an absorbent sheet which faces the backing sheet on the imprinted side. Another embodiment depicts the printing of the dye on the inside of the backing sheet in a predetermined pattern. U.S. Patent 4,192,311 provides a wetness indicator which is masked by an agent which when dry, masks the visibility of the indicator through the sheet, but when wet, unmasks the indicator. U.S. Patent 4,231,370 provides a wetness indicator which is disposed intermediate the translucent cover member and the absorbent member and which is a coating consisting of a solid-solid mixture of a pH change/color-change type material dispersed in a highly flexible matrix of adhesive.

In each of the products discussed above, in order to provide a wetness indicator, either a rather complicated registry system is necessary during manufacturing or another element needs to be added to the overall product or requires that the wetness indicator not be associated with the absorbent pad.

### Summary of the Invention

The present invention provides a disposable diaper which has an absorbent layer sandwiched between a liquid-permeable facing and a liquid-impermeable backing which is light transmitting or translucent. The absorbent layer comprises a loosely-compacted, cellulosic fibrous batt having a paper-like densified skin on one side. The paper-like densified skin is placed adjacent the backing sheet. This densified skin contains a water-dispersible wetness indicator in a pattern which is discernible through the backing sheet. The densified skin is an integral part of the loosely-compacted, cellulosic fibrous batt. This skin is provided by a method disclosed in U.S. Patent 3,017,304 to Burgeni. The Burgeni patent is incorporated herein by reference. The wetness indicator composition of the present invention is water-dispersible or water-soluble or both.

In manufacturing the product of the present invention, the densified skin is formed prior to application of the wetness indicator. One method of applying the wetness indicator is to spray a line approximately 1/4 inch in width, of the wetness indicator onto the backing sheet and immediately placing the backing sheet against the densified skin side of the absorbent batt whereupon the wetness indicator transfers immediately to the batt. Another method is applying the wetness indicator in the desired predetermined pattern directly onto the densified skin of the absorbent batt after which the backing sheet is placed against the densified skin side of the batt.

### Brief Description of the Drawings

Figure 1 is a perspective view of an embodiment of the present invention ;
Figure 2 is an exploded perspective view showing relative positioning of diaper elements during manufacture of the diaper shown in Figure 1 ;

Figure 3 is a cross-sectional view of a fragment taken along line 3-3 of Figure 2 ; and

Figure 4 is a perspective view of another embodiment of the present invention.

## Detailed Description of the Invention

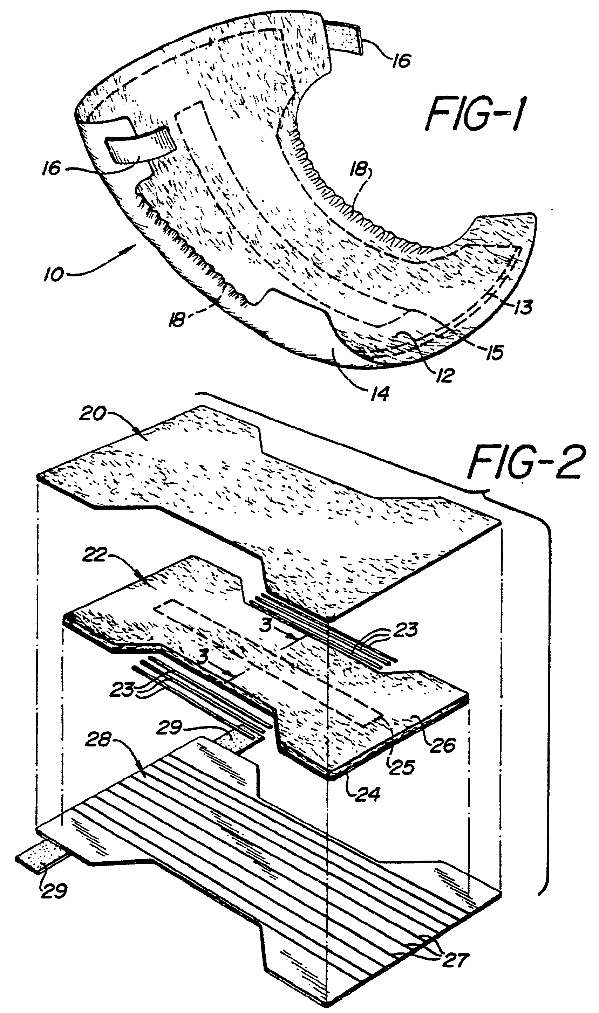

Figure 1 depicts a disposable diaper 10 having a liquid permeable facing 12, a light-transmitting or translucent backing sheet 14, and an absorbent layer 13 sandwiched between the facing 12 and the backing sheet 14. The absorbent layer 13 has a wetness indicator strip 15 running longitudinally of the diaper substantially in the center. The diaper 10 has elastic regions 18 on each side of the diaper in the central portion. Tape tabs 16 secure the diaper about the waist of the wearer.

Figure 2 is an exploded view of the diaper in Figure 1 showing each of the component parts. The facing 20 is of a liquid-permeable fabric, such as a high-loft polyester fabric. The absorbent layer 22 is a loosely-compacted, cellulosic fibrous batt having a densified skin layer 26 on one side of the batt. The other side of the batt is made of loosely-compacted cellulosic fibers, such as wood pulp fiber. A wetness indicator dispersed in liquid, is placed in contact with the densified skin which substantially absorbs and retains the wetness indicator 25. The densified skin 26 is relatively thin compared to the overall thickness of the absorbent batt 24, but it retains the pattern of the wetness indicator 25 without any substantial portion of the wetness indicator extending into the loosely-compacted portion of the fibrous batt. Elastic bands 23 are shown in the approximate position in which they would be placed when extended and secured into the margin of the diaper. The liquid-impermeable, light-transmitting or translucent backing sheet 28 has placed thereon glue lines 27 to which the other diaper components are then adhered to complete the unity of the overall diaper product. Tape tabs 29 are generally secured to the backing sheet 28 and may also or exclusively be secured to the facing 20.

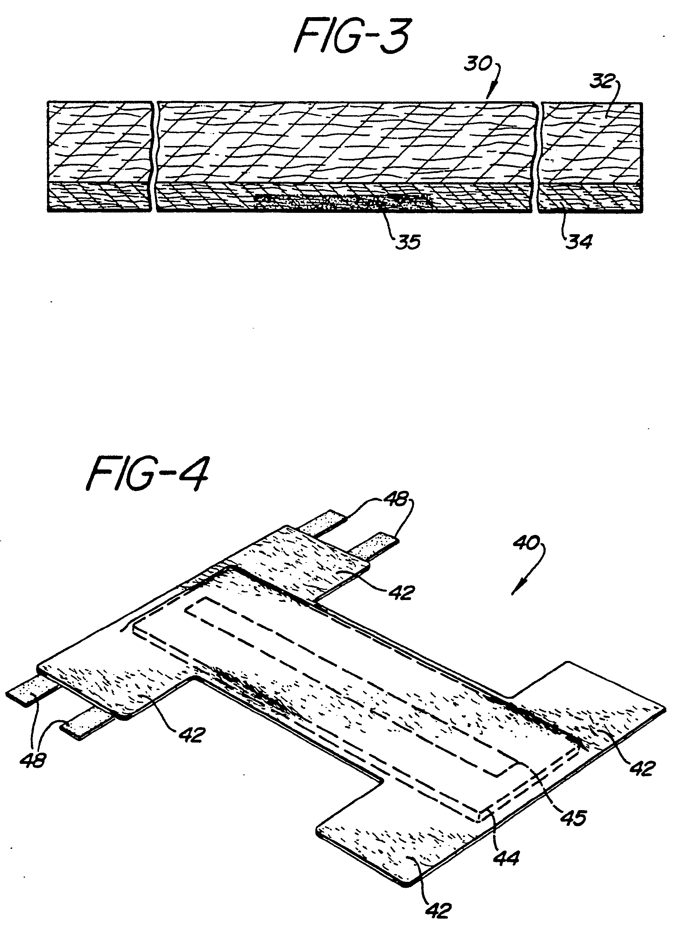

Figure 3 is a cross-sectional view of a fragment of the loosely-compacted cellulosic fibrous batt containing a densified skin. The batt 30, as shown on the upper surface, has a loosely compacted portion 32 and on the lower portion of the batt a densified skin 34. The densified skin 34 substantially retains all of the wetness indicator 35 which has been placed on the densified skin surface.

Figure 4 shows an adult diaper 40 which is made substantially in the configuration of an "I". The diaper has four fabric wings 42 which extend beyond the central portion which contains the absorbent batt 44. The absorbent batt 44 is made up of a liquid-impermeable backing surface, a loosely-compacted, cellulosic fibrous batt with a densified skin, and a wetness indicator 45. The upper surface of the absorbent layer 44 is covered by a facing which is liquid permeable. The product is secured about the waist of the wearer by two pairs of tape tabs 48.

It has been discovered that by placing a water-dispersible or water-soluble wetness indicator composition on a densified skin of a loosely-compacted, cellulosic fibrous batt, when the batt becomes wet, the pattern of the indicator is very rapidly dispersed and appears to disappear throughout the densified skin region. It is preferred that the indicator pattern is placed primarily in the central portion of the densified skin region for two reasons. First of all the liquid will reach the pattern quickly ; and therefore, the dispersion and disappearing of the indicator warns a caretaker of the need for the change of the product. Further, the indicator will disappear more rapidly if the surrounding regions are of the densified skin permitting rapid distribution of the indicator throughout the skin area. If the indicator pattern is placed too close to the edge of the product, there may not be sufficient densified skin area for the indicator to disperse within so as to quickly disappear upon contact with liquid.

Although it is not necessary to adhere the densified skin of the fibrous batt to the interior of the backing sheet, it is desirable that the surface of the densified skin remain in close association with the backing sheet so that the view of the indicator pattern through the backing sheet remains clear until it is dispersed when the product is used.

Suitable backing sheet materials include polyethylene films, polypropylene films, or other film material which permits sufficient light to be transmitted through the film for the indicator pattern to be substantially clearly visible.

Suitable cellulosic fibers for the cellulosic fibrous batt include wood pulp fibers, cotton linters, mixtures thereof and the like.

Suitable fibrous structures for making the batts of the present invention are made from short cellulosic fibers obtained by the defiberizing or comminution of wood pulp board or fiber bales. The compacted cellulosic material is at a moisture content of 5-10 percent by weight or is slightly moistened to bring it to that range before being subjected to the defiberizing operation so that the fibers produced by defiberizing have sufficient moisture to have the capability of developing weak intra fiber hydrogen bonds which give some coherence to the body of the batt.

The batts generally are initially formed by air laying the slightly moist cellulosic fibers onto a support to provide a weight of about 2 to about 15 or more oz/yd$^2$ and then subjected to compression. The small amount of moisture present in the cellulosic fibers is uniformly distributed throughout the air laid fibers by the defiberizing and air laying operations and after compression the moisture provides weak hydrogen bonding to give some integrity to the body of the batt.

The dense compacted paper-like layer of skin is prepared by moistening a surface of the cellulosic batt with a fine spray of water and then subjecting the moistened batt to pressure. The formation of the densified skin on the cellulosic batt is believed to be due to the formation of strong hydrogen bonds between moistened fibers which are in contact with each other similar to the bonds between the fibers in paper. By the proper selection of the amount of moisture applied to the surface of the batt and by the proper selection of degree of compression imposed, the properties of the densified skin may be varied as desired. The thickness, density, strength, and other characteristics of the densified skin will depend upon the uniformity by which the moisture is applied, the depth to which it penetrates, and the degree to which the fibers are compressed. For example, by finely spraying about 0.0015 cc of water per square centimeter of batt surface and then exposing the web to a pressure of about 40 psi, a suitable densified coherent paper-like skin is obtained on the surface of the batt which has been moistened.

The short fibers used in making batts suitable for use in the present invention, are generally entirely fibers of wood pulp or cotton linters ; however, other cellulosic fibers may be used as well as blends of cellulose fibers with other fibers, such as silk, wool, nylon, polyester, polypropylene, cellulose acetate, and the like.

The paper-like densified skin can also be provided by a layer of tissue substantially adhered to one surface of the batt so as to be substantially integral with the batt.

The amount of moisture applied to the batt may vary suitably from about 0.0005 to about 0.03 cc of water per square centimeter of batt surface. Depending on the thickness of the batt and the thickness of the paper-like densified skin desired with lesser amounts of moisture being used for thinner batts and very thin papery skins and greater amounts for thicker batts and skins of greater thickness.

The amount of pressure applied to compress the batt after moisture is applied may vary from about 5 to about 100 or more psi with the preferred range being from about 40 to about 50 psi. In a typical embodiment the batt is sprayed with about 0.0015 cc of water per square centimeter of batt surface and subjected to a pressure of about 10 psi to obtain a densified coherent papery skin on the surface of the batt which has been moistened.

The facing layer generally is made from a nonwoven fabric of a substantially synthetic hydrophobic fiber, such as polyester fibers, polypropylene fibers and the like. The facing fabric is sufficiently wettable for liquid to pass through but liquid preferably is not retained by the facing fabric.

An important aspect of the present invention is the provision for selective wettability among the above-described fibrous components of the diaper product such that the moisture is selectively drawn from the facing layer into the body of the batt and then from the body of the batt into the densified skin layer.

The least wettable of the fibrous elements of the diaper is the facing layer.

A useful parameter for determining the desired degree of wettability of the fibers of the facing is the liquid-fiber contact angle for the individual fibers of the layer. The contact angle approaching 90° depicts fibers which are difficultly wetted. When exceeding 90° the fibers are highly water repellant and when approaching 0°, the fibers are highly wettable by water. The liquid-fiber contact angle may be determined from interface high speed photographs. The individual dry fibers are held in a clamp and advanced into the wetting liquid (water) at a rate of 0.5 cm/sec by techniques known in the art.

In any particular facing material a liquid-fiber contact angle for individual fibers may vary considerably because of unevenness of distribution of bonding agents, thermal bonding distribution of any wetting agents, and the like. Nevertheless, a liquid-fiber contact angle between about 30° and about 60° for most of the individual fibers in a random selection provide suitable wettability in the facing layer and a liquid-fiber contact angle between about 40° and about 60° is preferable.

The body of the batt is substantially more wettable than the facing layer and tends to draw liquid away from the facing layer. The individual fibers of the batt are extremely wettable, generally having liquid-fiber contact angles below about 15° and approaching 0° in the optimum embodiment. The wickability or preferential absorptivity of the body of the batt for water is limited, however, by its low density which results in a large effective capillary radius with the capillaries between adjacent fibers.

The pressure causing the liquid to enter a cylindrical capillary is expressed by the equation :

$$P = \frac{(2r \cos \theta)}{r}$$

P is the capillary pressure
r is the surface tension of the liquid
θ is the liquid-fiber contact angle, and
r is the capillary radius.

With a given liquid the pressure (capillary force) increases with the cosine of the liquid-fiber contact angle (reaching a maximum or the angle is 0) and increases with narrower capillary radii so that narrower capillaries will draw liquid from wider ones.

The relative wickability between the facing layer and the absorbent batt is affected by both the relative densities of the layers and the relative wettability of the individual fibers in each layer. The facing layer generally is less dense than the body of the batt and the individual fibers of the batt have substantially smaller liquid-fiber contact angles than those of the facing. Thus, liquid tends to pass easily through the facing and into the batt. Even if the facing layer is sometimes more dense than the batt layer, the individual fibers of the batt layer with their high degree of wettability will overcome the density difference and provide a substantial overall increase in capillary pressure to absorb liquid into the body of the batt. The densified fiber layer, i.e., the densified paper-like skin of the batt, provides the maximum capillary pressure because it combines the very low contact angle of the batt fibers with the high density (small capillary radius) of the densified fibers.

Hence, in the present invention the liquid is transported rapidly to the densified layer containing the wetness indicator and since the densified layer attracts and transports liquid, the wetness indicator is sufficiently diluted so as to very rapidly disappear upon contact with liquid.

Examples typical of the present invention follow. These examples are are intended to be limiting in any way. Any extensions or modifications thereof without departure from the spirit and scope of the invention will become apparent from the examples.

## Example 1

An absorbent wood pulp fiber batt is prepared, the batt having an average density of about 0.11 g/cc and a total weight of about 70 grams with dimensions of about 10 inches by about 24 inches. A paper-like densified skin is formed on one side of the batt by spraying a water solution containing a minor amount of trisodium ethylenedianinetetraacetic acid on one surface of the batt so as to provide about 0.0015 cc/sq. in. of solution. The batt is then subjected to pressure in the range of 40 psi. A wetness indicator is prepared by mixing 0.227 gm of bromcresol purple per liter of 2-propanol. This mixture is sprayed at the rate of six gallons per hour on a polyethylene backing sheet to give a yellow line 1/4 inch wide. After formation of the densified skin, the batt is placed in contact with the backing sheet with the densified skin layer contacting the 1/4 inch line of wetness indicator. In this particular instance the indicator line, is transferred substantially in totality to the densified skin and changes to a purple color because the skin is alkaline due to the trisodium EDTA.

An inspection of the indicator line reveals that the line is 1/4 inch in width and approximately 1/32 inch in depth and is totally incorporated in the densified skin portion of the absorbent batt.

A facing sheet is added to complete the diaper product. Upon use the indicator line disappears within seconds after the diaper becomes wet. Other dye solutions may be used provided that the dye is water-dispersible or water-soluble or both and is in sufficient concentration to provide a predetermined pattern visible through a translucent or light transmitting backing sheet of the diaper product.

## Example 2

An absorbent batt is prepared in accordance with Example 1 except that the batt weighs about 25 grams and is shaped in an hour-glass shape with overall dimensions of about 14 inches long and 10 inches wide, and no trisodium EDTA is present.

A wetness indicator solution of about 0.3 gm of Acid Blue 80 in one liter of 2-propanol is sprayed as in Example 1 on the backing sheet of the infant diaper. The densified skin is brought into contact with the backing sheet and as before the indicator solution transfers to the densified skin.

A facing is added and the diaper is glued together to provide an unitized product.

When the diaper used, the indicator disappears in a matter of seconds as the water-dispersible dye is diluted and quickly transported throughout the densified skin region.

One advantage of the present invention is the ability to color code diaper products as to size and/or type by use of different colored dye indicators. Since the indicator merely disappears and there is no color change involved, a wide variety of colors are available for color coding.

From the foregoing it will be observed that numerous variations and modifications may be effected without departing from the scope of the novel concept of this invention.

## Claims

1. A disposable diaper comprising an absorbent layer sandwiched between a liquid-permeable facing and a liquid-impermeable but light transmitting backing sheet, said absorbent layer comprising a loosely-compacted cellulosic fibrous batt having as an integral part thereof a paper-like densified skin on the side of the batt adjacent the backing sheet, said densified skin containing a water-dispersible wetness indicator in a predetermined pattern discernible through said backing sheet.

2. The disposable diaper of claim 1, wherein said densified skin is formed by spraying water in an amount from 0.0005 to 0.03 cc per square centimeter of surface onto one surface of said batt and subjecting said batt to pressure of about 40 psi.

3. The disposable diaper of claim 1 or claim 2, wherein said wetness indicator is bromcresol purple.

4. The disposable diaper of claim 1 or claim 2, wherein said wetness indicator is Acid Blue 80.

5. The disposable diaper of any one of claims 1 to 4, wherein said wetness indicator is applied to said densified skin as a stripe extending substantially the entire length of said batt.

## Ansprüche

1. Wegwerfwindel, bestehend aus einer absorbierenden Schicht zwischen einer flüssigkeitsdurchlässigen Vorderschicht und einer flüssigkeitsundurchlässigen, jedoch lichtdurchlässigen Rückschicht, wobei die absorbierende Schicht sich aus einem Schichtkörper zusammensetzt, der lose verdichtete Cellulosefasern enthält und als integralen Bestandteil eine papierähnliche verdichtete Haut auf derjenigen Seite des Schichtkörpers aufweist, die der Rückschicht benachbart ist, und die verdichtete haut einen in Wasser dispergierbaren Feuchtigkeitsindikator in einem vorbestimmten Muster enthält, das durch die Rückschicht erkennbar ist.

2. Wegwerfwindel nach Anspruch 1, in der die verdichtete Haut durch Aufsprühen von Wasser in einer Menge von 0,0005 bis 0,03 cm³ je cm² der Oberfläche auf eine Oberfläche des Schichtkörpers und Russetzen des Schichtkörpers einem Druck von etwa 40 psi hergestellt ist.

3. Wegwerfwindel nach Anspruch 1 oder 2, in der der Feuchtigkeitsindikator Bromcresol-Purpur ist.

4. Wegwerfwindel nach Anspruch 1 oder 2, in der der Feuchtigkeitsindikator acid Blue 80 ist.

5. Wegwerfwindel nach einem der Ansprüche 1 bis 4, in der der Feuchtigkeitsindikator auf die verdichtete Haut als im wesentlichen sich über die Gesamtlänge des Schichtkörpers erstreckender Streifen aufgebracht ist.

## Revendications

1. Une couche jetable pour bébés comprenant une couche absorbante intercallée entre une feuille interne perméable aux liquides et une feuille externe imperméable aux liquides mais permettant une légère transmission, ladite couche absorbante comprenant un molleton fibreux cellulosique lâchement compacté muni, en tant que partie intégrante, d'une pellicule densifiée semblable à du papier sur le côté du molleton adjacent à la feuille externe, ladite pellicule densifiée contenant un indicateur d'humidité dispersable dans l'eau disposé selon un motif prédéterminé visible au travers de ladite feuille externe.

2. La couche jetable pour bébés selon la revendication 1, dans laquelle ladite pellicule densifiée est formée en vaporisant une quantité d'eau allant de 0,0005 à 0,03 cm³ par cm² de surface, sur une surface dudit molleton et en soumettant ledit molleton à une pression d'environ 40 psi.

3. La couche jetable pour bébés selon la revendication 1 ou la revendication 2, dans laquelle ledit indicateur d'humidité est du bromocrésol pourpre.

4. La couche jetable pour bébés selon la revendication 1 ou la revendication 2, dans laquelle ledit indicateur d'humidité est de l'Acid Blue 80.

5. La couche jetable pour bébés selon l'une des revendications 1 à 4, dans laquelle ledit indicateur d'humidité est appliqué sur ladite pellicule densifiée sous la forme d'une bande s'étendant substantiellement sur la totalité de la longueur dudit molleton.

*FIG-1*

*FIG-2*

## FIG-3

## FIG-4